(19) Europäisches Patentamt
European Patent Office
Office européen des brevets

(11) Veröffentlichungsnummer: **0 182 298**
**B1**

(12) # EUROPÄISCHE PATENTSCHRIFT

(45) Veröffentlichungstag der Patentschrift: **06.12.89**

(51) Int. Cl.⁴: **C07C 172/00**, C07C 35/52, A61K 31/59

(21) Anmeldenummer: **85114493.1**

(22) Anmeldetag: **14.11.85**

(54) **Cholecalciferolderivate und ein Verfahren zu deren Herstellung.**

(30) Priorität: **16.11.84 US 672059**

(43) Veröffentlichungstag der Anmeldung: **28.05.86 Patentblatt 86/22**

(45) Bekanntmachung des Hinweises auf die Patenterteilung: **06.12.89 Patentblatt 89/49**

(84) Benannte Vertragsstaaten: **AT BE CH DE FR GB IT LI LU NL SE**

(56) Entgegenhaltungen:
**EP-A- 0 115 314**
**EP-A- 0 122 490**
**WO-A-83/00335**
**US-A- 4 521 410**

(73) Patentinhaber: **F. HOFFMANN-LA ROCHE AG, Postfach 3255, CH-4002 Basel(CH)**

(72) Erfinder: **Baggiolini, Enrico, 30 Evergreen Drive, North Caldwell, N.J. 07006(US)**
Erfinder: **Pizzolato, Giacomo, 194 Forest Avenue, Glen-Ridge, N.J. 07028(US)**
Erfinder: **Uskokovic, Milan, 253 Highland Avenue, Upper Montclair, N.J. 07043(US)**
Erfinder: **Truitt, Gary, 109 Garner Avenue, Bloomfield, N.J. 07003(US)**

(74) Vertreter: **Lederer, Franz, Dr. et al, Van der Werth, Lederer & Riederer Patentanwälte Lucile-Grahn-Strasse 22, D-8000 München 80(DE)**

**EP 0 182 298 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Cholecalciferolderivate, ein Verfahren zu ihrer Herstellung, neue in diesem Verfahren einsetzbare Zwischenprodukte und Medikamente auf der Basis der besagten Cholecalciferolderivaten.

Die erfindungsgemässen Cholecalciferolderivate sind das Δ²²-oder Δ²³-26,26,26,27,27,27-Hexafluor -1α,25-dihydroxycholecalciferol, im folgenden Δ²²-bzw. Δ²³-Verbindung genannt. Nach dem Verfahren der vorliegenden Erfindung werden diese Verbindungen dadurch hergestellt, dass man [1R-[1ß(R*),3aα,4ß,7aß]]- Octahydro-1-[6,6,6-trifluor -5-trimethylsilyloxy-5- trifluormethyl-1-methyl -2-hexenyl]-7a-methyl-1H-inden-4-on bzw. [1R-[1ß(R*),3aα,4ß,7aß]]-Octahydro-1-[6,6,6,-trifluor-5- trimethylsilyloxy-5-trifluormethyl-1-methyl-3-hexenyl] 7a-methyl-1H-inden-4-on mit [3S-(3α,5ß,Z)]-2-[2-Methylen- 3,5-bis[(1,1-dimethyläthyl)dimethylsilyloxy] cyclohexyliden]äthyldiphenylphosphixoxid behandelt und die Silylschutzgruppen abspaltet.

Diese Reaktion kann bei niedriger Temperatur durchgeführt werden, z.B. unterhalb von -50°C, vorzugsweise bei etwa -78°C, unter einer inerten Atmosphäre, z.B. unter Argon, in einem inerten Lösungsmittel, wie einem cyclischen Aether, vorzugsweise Tetrahydrofuran (THF). Zweckmässigerweise wird das Phosphinoxid zunächst in das entsprechende Carbanion umgewandelt. Dies kann man so durchführen, dass man zunächst das Phosphinoxid mit einem Alkyllithium, z.B. n-Butyllithium, in einem inerten Lösungsmittel, wie einem niederen Alkan, z.B. Hexan, bei reduzierter Temperatur behandelt. Das resultierende Produkt kann z.B. durch Chromatographie auf Silicagel gereinigt werden.

Im Rahmen der Erfindung bezieht sich der Ausdruck "Niederalkyl" auf gerad- oder verzweigtkettige Alkylgruppen mit 1-8 C-Atome, z.B. Methyl, Aethyl, n-Propyl, i-Propyl und t-Butyl. "Aryl" bedeutet Phenyl, gewünschtenfalls substituiert durch Alkyl, Halogen, Nitro, Cyan und Trifluormethyl. Beispiele von Aralkylgruppen sind Benzyl und Phenäthyl.

Die im obigen Verfahren verwendeten Ausgangsindenone können wie folgt hergestellt werden:

[1R-[1ß,[αS*,ßS*],3aα,4aß,7aß]]-Octahydro-ß,7a- dimethyl -4-[(1,1-dimethyläthyl)dimethylsilyloxy]-α-äthenyl-1H-inden-1-äthanol kann durch Behandlung mit Thionylchlorid gefolgt von Pyridin in [1R-[1ß(R*),3aα,4ß,7aß]]-1- (4-Chlor-1-methyl-2-butenyl)octahydro -4-[(1,1-dimethyläthyl)dimethylsilyloxy]-7a-methyl -1H-inden übergeführt werden. Das resultierende allylische Chlorid wird dann mit einem Arylsulfinsäuresalz, z.B. dem Benzolsulfinsäurenatriumsalz, zur entsprechenden 4-Arylsulfonylverbindung, z.B. [1R-[1ß(R*),3aα,4ß,7aß]] -1-[(4-Phenylsulfonyl)- 1-methyl-2-butenyl]octahydro -4-[(1,1-dimethyläthyl)dimethylsilyloxy]-7a-methyl -1H-inden umgesetzt. Zur Vollendung der Seitenkette in einer erhaltenen Sulfonylverbindung kann man letztere mit n-Butyllithium oder Lithiumdiisopropylamid und das entstandene Carbanion mit Hexafluoraceton umsetzen.

In der erhaltenen Verbindung der Formel

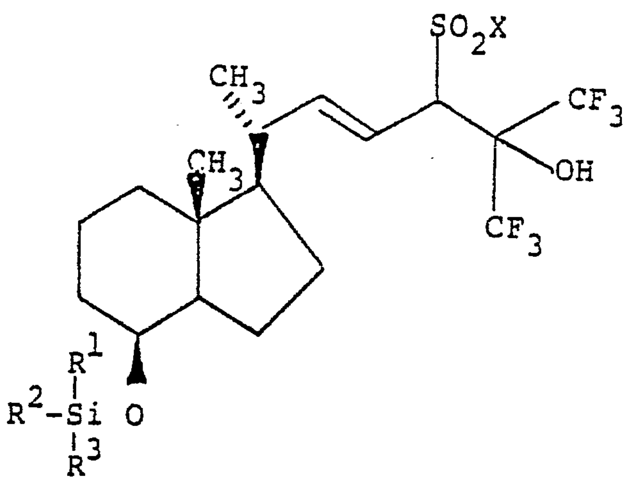

I

worin X Aryl, vorzugsweise Phenyl, $R^1$ und $R^3$ Methyl und $R^2$ t-Butyl sind.

Die Verbindung der Formel I kann zunächst mit einem Di-alkalimetallphosphat, z.B. Dikaliumhydrogenphosphat, zweckmäßigerweise unter Raumbedingungen, und dann mit einem Alkalimetallamalgam, z.B. Natriumamalgam, zweckmäßigerweise bei einer Temperatur unterhalb von 0°C, z.B. bei etwa –20°C, umgesetzt werden. Die Reaktion kann in einem inerten Lösungsmittel, wie einem niederen Alkanol, z.B. Methanol, einem cyclischen Äther, z.B. THF, vorzugsweise in Gemischen davon, durchgeführt werden. Die Reaktionsprodukte [1R-[1β(R*),3aα,4β,7aβ]]-Octahydro-1-[6,6,6-trifluor-5-hydroxy-5-trifluormethyl-1-methyl-2-hexenyl]-7a-methyl-1H-inden-4-ol oder [1R-[1β(R*),3aα,4β,7aβ]]-Octahydro-1-[6,6,6-trifluor-5-hydroxy-5-trifluormethyl-1-methyl-3-hexenyl]-7a-methyl-1H-inden-4-ol können in gereinigter und getrennter Form erhalten werden durch eine erste Chromatographie auf einer Silicagelsäule gefolgt durch

Behandlung mit einem Kationenaustauscherharz und schließlich durch selektive Chromatographie auf einer Silicagelsäule.

In der nächsten Stufe kann man ein erhaltenes Indenolderivat (Verbindung II) mit einem basischen organischen Aminchromatsalz, z.B. Pyridiniumhalochromat, vorzugsweise Pyridiniumchlorochromat behandeln. Die Reaktion wird zweckmäßigerweise unter Raumbedingungen, in einem inerten Lösungsmittel, wie einem halogenierten Alkan, z.B. einem Chloralkan, vorzugsweise Methylenchlorid, durchgeführt.

Ein erhaltenes Indenonderivat (Verbindung III) kann dann bei Raumtemperatur unter einer inerten Atmosphäre, z.B. unter Argon, mit einem eine Trimethylsilylschutzgruppe in die Hydroxygruppe der Seitenkette einführenden Mittel, z.B. Trimethylsilylimidazol, behandelt werden.

Die erhaltenen Verbindungen [1R-[1β(R*),3aα,4β,7aβ]]-Octahydro-1-[6,6,6-trifluor-5-trimethylsilyloxy-5-trifluormethyl-1-methyl-2-hexenyl]-7a-methyl-1H-inden-4-on oder [1R-[1β(R*),3aα,4β,7aβ]]-Octahydro-1-[6,6,6-trifluor-5-trimethylsilyloxy-5-trifluormethyl-1-methyl-3-hexenyl]-7a-methyl-1H-inden-4-on sind neu und als solche Gegenstand der Erfindung.

Die $\Delta^{22}$- und $\Delta^{23}$-Verbindungen haben Vitamin $D_3$-ähnliche Aktivität und zeigen antiproliferative und die Zelldifferenzierung induzierende Effekte. Diese Effekte auf HL-60-Zellen in vitro können nach an sich bekannten Methoden, z.B. wie beschrieben in Progress in Cancer Research and Therapy, Vol. 23: Maturation Factors and Cancer, Ed. M.A.S. Moore, Raven Press, N.Y. 1982; Nature 270 (1977) 347–9 and Blood 54 (1979) 429–39 demonstriert werden. Die Resultate sind in folgender Tabelle wiedergegeben:

| Konzentration der [a] $\Delta^{22}$-Verbindung ($\times 10^{-9}$ Molar) | Proliferation[b] HL-60-Zellen per ml $\times 10^{-4}$ | % Reduktion der Anzahl Zellen | Differentiation NBT-Reduktion Formazan "+" Summe der gez. Zellen | % "+" | Phagocytose; phagocytische Zellen Summe der gez. Zellen | % "+" |
|---|---|---|---|---|---|---|
| Experiment 1 | | | | | | |
| Null (Mediumkontrolle) | 77.1 ± 3.3 | – | 3/378 | <1 | | |
| Vehikel (0.1% Aethanol) | 76.9 ± 5.1 | 0 | 2/359 | <1 | | |
| 0.1 | 66.7 ± 1.6 | 13 | 6/347 | 2 | | |
| 1 | 24.8 ± 0.6 | 68 | 280/328 | 85 | | |
| 10 | 17.0 ±1.6 | 78 | 306/319 | 96 | | |
| 100 | 16.4 ± 1.1 | 79 | 348/356 | 98 | | |
| Experiment 2 | | | | | | |
| Null (Mediumkontrolle) | 92.2 ± 7.6 | – | 2/346 | <1 | 4/386 | 1 |
| Vehikel (0.1% Aethanol) | 94.1 ± 3.4 | 0 | 3/381 | <1 | 3/336 | <1 |
| 0.1 | 97.9 ± 6.2 | 0 | 3/343 | <1 | 4/359 | 1 |
| 0.3 | 74.3 ± 0.8 | 21 | 45/355 | 13 | 49/376 | 13 |
| 1 | 47.6 ± 1.3 | 49 | 212/317 | 67 | 229/330 | 69 |
| 3 | 30.9 ± 1.2 | 67 | 290/316 | 92 | 284/303 | 94 |
| 10 | 28.2 ± 0.6 | 70 | 353/360 | 98 | 311/328 | 95 |
| 30 | 25.3 ± 2.0 | 73 | 310/316 | 98 | 339/344 | 98 |
| Experiment 3 | | | | | | |
| Null (Mediumkontrolle) | 74.4 ± 1.9 | – | 2/346 | <1 | | |
| Vehikel (0.1% Aethanol) | 80.6 ± 4.8 | 0 | 3/374 | <1 | | |
| 0.1 | 74.4 ± 1.3 | 8 | 2/380 | <1 | | |
| 0.3 | 60.9 ± 2.0 | 24 | 34/338 | 10 | | |
| 1 | 38.4 ± 1.6 | 52 | 231/332 | 70 | | |
| 3 | 24.7 ± 1.0 | 69 | 354/367 | 96 | | |
| 10 | 21.0 ± 0.4 | 74 | 314/332 | 95 | | |
| 30 | 21.0 ± 0.5 | 74 | 312/322 | 96 | | |

| Konzentration der [a] $\Delta^{23}$-Verbindung ($\times 10^{-9}$ Molar) | | | | | | |
|---|---|---|---|---|---|---|
| Experiment 1 | | | | | | |
| Null (Mediumkontrolle) | 74.0 ± 1.0 | – | 3/318 | <1 | 11/350 | 3 |
| Vehikel (0.1% Aethanol) | 67.8 ± 0.5 | 0 | 3/316 | <1 | 7/345 | 2 |
| 0.1 | 64.8 ± 1.7 | 4 | 3/310 | <1 | 4/448 | <1 |
| 0.3 | 58.8 ± 3.6 | 13 | 16/351 | 5 | 12/343 | 4 |
| 1 | 42.0 ± 2.1 | 38 | 184/328 | 56 | 155/352 | 44 |
| 3 | 22.5 ± 1.4 | 67 | 342/360 | 95 | 305/330 | 92 |
| 10 | 16.2 ± 0.7 | 76 | 353/359 | 98 | 335/347 | 97 |
| 30 | 14.5 ± 0.8 | 79 | 335/339 | 99 | 372/379 | 98 |
| 100 | 13.6 ± 0.9 | 80 | 331/335 | 99 | | |
| Experiment 2 | | | | | | |
| Null (Mediumkontrolle) | 74.4 ± 1.9 | – | 2/343 | <1 | | |
| Vehikel (0.1% Aethanol) | 80.6 ± 4.8 | 0 | 3/374 | <1 | | |
| 0.1 | 71.4 ± 1.2 | 11 | 3/351 | <1 | | |
| 0.3 | 64.8 ± 7.3 | 20 | 13/327 | 4 | | |
| 1 | 55.1 ± 0.9 | 32 | 218/347 | 63 | | |
| 3 | 47.5 ± 3.0 | 41 | 284/324 | 88 | | |
| 10 | 44.0 ± 0.9 | 45 | 318/324 | 98 | | |
| 30 | 39.7 ± 2.2 | 51 | 346/354 | 98 | | |

a. In allen Testkulturen betrug die Vehikelkonzentration 0,1% v/v Aethanol.
b. In allen Testkulturen war die Zellenviabilität grösser als 95%. Die Anfangskonzentration aller Kulturen betrug $2 \times 10^{-4}$ HL-60-Zellen per ml.

Die Daten in der Tabelle zeigen, daß die $\Delta^{22}$- und $\Delta^{23}$-Verbindung die Proliferation der menschlichen promyelocytischen Tumorzellen in vitro hemmen und dabei nicht zellentoxisch sind. Ferner konnte man Zellen, die in niedrigen Konzentrationen dieser Verbindungen (0,3 bis $10 \times 10^{-9}$ Molar) gezüchtet worden waren, dazu befähigen, zu einem reiferen Zelltyp zu differenzieren, was sich durch Erlangen von Enzymaktivität und Zellfunktion äußerte. Es kann erwartet werden, dass die $\Delta^{22}$- und $\Delta^{23}$-Verbindungen bei der Behandlung von Krankheiten verwendet werden können wie neoplastische Krankheiten, die teilweise durch aberrierende Zellproliferation und/oder -differentiation verursacht sind.

Von dem in der EP-A 122 490 beschriebenen nicht fluorierten 1a,25-Dihydroxyergocalciferol und dem in der WO-A 83/335 beschriebenen in 22- und 23-Stellung gesättigten 26,26,26,27,27,27-Hexafluor-1a,25-Dihydroxycholecalciferol ist eine antiproliferative und die Zelldifferenzierung induzierende Wirksamkeit nicht bekannt.

Die $\Delta^{22}$- und $\Delta^{23}$-Verbindungen kann man in Dosierungen im Bereich von etwa 0,10–3,0, vorzugsweise 0,25–2,0 µg/Tag verabreichen zur Behandlung von Krankheiten, wie Osteoporose, Osteodystrophie, durch Steroide induzierte Osteopenie, Hypoparathyroidismus, hypophosphatämische Rachitis und hypophosphatämische Osteomalazie, die durch subnormale Spiegel an endogen produziertem 1α,25-Dihydroxycholecalciferol charakterisiert sind. Die $\Delta^{22}$- und $\Delta^{23}$-Verbindungen können im obigen Dosierungsbereich zur Behandlung von proliferativen Krankheiten wie Leukämie verabreicht werden. Die $\Delta^{22}$- und $\Delta^{23}$-Verbindungen kann man oral, z.B. in Form von Tabletten, Kapseln oder Elixier; oder z.B. in Form von sterilen Lösungen oder Suspensionen, subkutan, intramuskular, intravenös, intraperitoneal oder topisch verabreichen. Eine Einzeldosierung kann von etwa 0,10–3,0, vorzugsweise 0,25–2,0 µg der $\Delta^{22}$- oder $\Delta^{23}$-Verbindung enthalten, zusammen mit einem pharmazeutisch akzeptablen Vehikel, Träger, Konservierungsmittels, Stabilisierungsmittels, Bindemittels, z.B. Tragantgummi, Excipient, z.B. Kalziumphosphat, Sprengmittel, z.B. Maisstärke, Gleitmittel, z.B. Magnesiumstearat, Süßstoff, z.B. Sucrose, Geschmacksmittel, z.B. Pfefferminz. Es können andere Materialien, wie Überzüge, z.B. Shellac, zur Modifizierung des Aussehens der Einzeldosen verwendet werden.

Die $\Delta^{22}$- und $\Delta^{23}$-Verbindungen können auch zur Behandlung von Milchfieber bei trächtigen Wiederkäuern in Dosierungen im Bereich von 100-1500, vorzugsweise 200-1000 µg/Tag in herkömmlichen Formulierungen verabreicht werden. Zum Beispiel kann man sterile Präparate für Injektionen und/oder topische Verabreichung durch Auflösen oder Suspendieren der $\Delta^{22}$- oder $\Delta^{23}$-Verbindung in einem Vehikel, z.B. einer 10-20% oder 80-95% Aethanol (oder Propylenglykol)-Wassergemisch, einem in der Natur vorkommenden pflanzlichen Oel, wie Sesamöl, oder einem synthetischen fetten Bindemittel, wie Aethyloleat, herstellen. Die $\Delta^{22}$- oder $\Delta^{23}$-Verbindung lässt sich auch in einer Menge von 100-1500 µg in Form von Fettsäurepellets oral verabreichen.

Beispiel 1

a) Eine Lösung von 2,9 g (8,22 mMol)[1R-[1ß,[αS*,ßS*], -3aα,4aß,7aß]]-Octahydro-ß,7a-dimethyl -4-[(1,1-dimethyläthyl)dimethylsilyloxy] -α-äthenyl-1H-inden-1-äthanol in 100 ml wasserfreiem Aether wurde auf 0°C abgekühlt und tropfenweise unter Argon mit 2,76 ml (37,84 mMol) Thionylchlorid, gefolgt durch 0,276 ml Pyridin behandelt. Das Gemisch wurde 2 Stunden bei 0°C gerührt und dann mit einer Lösung von 50 ml einer 2N Lösung von Natriumkaliumtartrat versetzt. Die Aetherphase wurde abgetrennt und die wässrige Phase wurde mit Aethylacetat extrahiert. Die vereinigten organischen Phasen wurden mit 1N Salzsäure, Wasser, 2N Kaliumbicarbonat und Salzwasser gewaschen, getrocknet und eingedampft. Das Lösungsmittel wurde in Vakuum abgedampft und der Rückstand wurde durch Chromatographie auf Silicagel mit Hexanäthylacetat gereinigt. Man erhielt 2,9 g (95% Ausbeute) reines [1R-[1ß(R*),-3aα,4ß,7aß]]-1-(4-Chlor-1-methyl-2-butenyl)octahydro-4-[(1,1-dimethyläthyl)dimethylsilyloxy]-7a-methyl-1H-inden in Form eines tiefschmelzenden Feststoffs.

b) Eine Lösung von 2,9 g (7,81 mMol) des allylischen Chlorids von 1a) in 130 ml Hexamethylphosphoramid wurde mit 10,1 g (61,52 mMol) Benzolsulfinsäurenatriumsalz behandelt und bei Raumtemperatur unter Argon 24 Stunden gerührt. 130 ml Eiswasser wurden dann zugesetzt und nach Rühren während 30 Minuten wurde das Gemisch mit Aethylacetat extrahiert. Die vereinigten Extrakte wurden mit Wasser gewaschen, getrocknet und zur Trockene eingedampft. Der Rückstand wurde durch Chromatographie auf Silicagel mit Hexan-Aethylacetat gereinigt. Man erhielt 3,5 g (94% Ausbeute) [1R-[1ß(R*),3aα,4ß,7aß]]-1-[(4-Phenylsulfonyl)-1-methyl-2-butenyl]octahydro-4-[(1,1-dimethyläthyl)dimethylsilyloxy]-7a-methyl-1H-inden, in Form eines tiefschmelzenden Feststoffs.

c) Eine Lösung von 0,628 ml (4,48 mMol) Diisopropylamin in 10 ml wasserfreiem THF wurde auf 0°C abgekühlt und tropfenweise unter Argon mit 2,70 ml (4,32 mMol) einer 1,6 molaren Lösung von n-Butyllithium in Hexan behandelt. Nach Rühren während 15 Minuten bei 0°C wurde die resultierende Lösung auf -78°C abgekühlt und mit 17 ml wasserfreiem THF verdünnt. Es wurde dann tropfenweise mit einer Lösung von 1,25 g (2,62 mMol) des Sulfons von 1b) in 16 ml THF behandelt und 30 Minuten bei -78°C gerührt. Hexafluoraceton wurde durch die Lösung eingeblasen bis zum Verschwinden der gelben Färbung. Nach Rühren während 5 Minuten wurde das Reaktionsgemisch durch Zusatz von 30 ml eines 1:1 Gemisches von 2N Natriumkaliumtartrat und 2N Kaliumbicarbonat abgekühlt, dann auf Raumtemperatur Aufwärmen gelassen und mit Methylenchlorid extrahiert. Die organischen Extrakte wurden mit Salzwasser gewaschen, getrocknet und zur Trockene eingedampft. Der Rückstand wurde durch Chromatographie auf Silica mit Hexan-Aethylacetat gereinigt. Man erhielt 1,23 g (72%) Ausbeute [1R-[1ß(R*),3aα,4ß,7aß]]-1-Octahydro-1-[6,6,6-trifluor-5-hydroxy-5-trifluormethyl-4-phenylsulfonyl-1-methyl-2-hexenyl-4-[(1,1-dimethyläthyl)-dimethylsilyloxy]-7a-methyl-1H-inden in Form eines farblosen Oels.

d) Eine Lösung von 1,23 g (1,91 mMol) des Sulfons von 1c) in Form eines epimerischen Gemisches in 40 ml Methanol und 40 ml THF wurde mit 23 g Dikaliumhydrogenphosphat und nach Abkühlen auf -20°C mit 24 g 6% Natriumamalgam behandelt. Nach Rühren des resultierenden Gemisches während 15 Minuten wurden 60 ml Salzwasser zugesetzt. Nach Aufwärmen auf Raumtemperatur wurde mit Aethylacetat extrahiert. Die organischen Extrakte wurden mit Salzwasser gewaschen, getrocknet und zur Trockene eingedampft. Der Rückstand wurde durch Chromatographie auf Silicagel mit Hexan-Aethylacetat gereinigt. Das Produkt (0,815 g) wurde in 40 ml Aethanol gelöst, bei Raumtemperatur mit 10 g eines Kationaustauscherharzes (AG 50W-X4, 200-400 Mesh, Bio-Rad Laboratories, Richmond, CA) 6 Tage gerührt. Nach Filtrierung des Harzes und Abdampfen des Lösungsmittels wurde der Rückstand durch zwei nacheinanderfolgenden Chromatographien auf Silicagel, die erste mit Hexan-Aethylacetat und die zweite mit Methylenchlorid, gereinigt. Man erhielt 200 mg reines [1R-[1ß(R*),3aα,4ß,7aß]]-Octahydro-1-[6,6,6-trifluor-5-hydroxy-5-trifluormethyl-1-methyl-2-hexenyl]-7a-methyl-1H-inden-4-ol und 50 mg [1R-[1ß(R*),3aα,-4ß,7aß]]-Octahydro-1-[6,6,6-trifluor-5-hydroxy-5-trifluormethyl-1-methyl-3-hexenyl]-7a-methyl-1H-inden-4-ol.

e) Eine Lösung von 182 mg (0,469 mMol) des Diols [1R-[1ß(R*),3aα,4ß,7aß]]-Octahydro-1-[6,6,6-trifluor -5-hydroxy-5-trifluormethyl-1-methyl -2-hexenyl]-7a-methyl-1H-inden-4-ol in 2 ml Methylenchlorid wurde einer Aufschlämmung von 300 mg (1,392 mMol) Pyridiniumchlorochromat in 7 ml Methylenchlorid zugesetzt. Das resultierende Gemisch wurde 2 1/2 Stunden bei Raumtemperatur gerührt. Es wurde mit 10 ml Aether verdünnt, 15 Minuten gerührt und filtriert. Der Rückstand wurde mehrmals mit Aether verrieben. Die Triturationsextrakte wurden kombiniert und filtriert. Nach Eindampfen zur Trockene und Reinigung des Rückstandes durch Chromatographie mit Hexan-Aethylacetat erhielt man 174 mg (96% Ausbeute) reines [1R-[1ß(R*),3aα,4ß,7aß]]-Octahydro-1-[6,6,6-trifluor-5-hydroxy-5-trifluormethyl-1-methyl-2-hexenyl]-7a-methyl-1H-inden-4-on.

f) Eine Lösung von 174 mg (0,450 mMol) des Ketons von le) in 9 ml Methylenchlorid wurde mit 0,4 ml

(2,726 mMol) Trimethylsilylimidazol behandelt und 6 Stunden bei Raumtemperatur unter Argon gerührt. Nach Zusatz von 1 ml Wasser wurde das Gemisch 20 Minuten gerührt, dann mit Wasser verdünnt und mit Aethylacetat extrahiert. Die organischen Extrakte wurden mit Wasser und Salzlösung gewaschen, getrocknet und zur Trockne eingedampft. Der Rückstand wurde durch Chromatographie mit Hexan-Aethylacetat gereinigt. Man erhielt 177 ml (86% Ausbeute) reines [1R-[1ß(R*),3aα,4ß,7aß]]-Octahydro-1-[6,6,6-trifluor -5-trimethylsilyloxy-5-trifluormethyl-1-methyl -2-hexenyl]-7a-methyl-1H-inden-4-on.

g) Eine Lösung von 365 mg (0,584 mMol)[3S-(3α,5ß,Z)]-2-[2-methylen-3,5-bis[(1,1-dimethyläthyl)dimethylsilyloxy] cyclohexyliden]äthyldiphenylphosphinoxid in 10 ml wasserfreiem THF wurde auf -78°C abgekühlt und tropfenweise unter Argon mit 0,358 ml (0,573 mMol) einer 1,6 molaren Lösung von n-Butyllithium in Hexan behandelt. Nach Rühren während 5 Minuten wurde eine Lösung von 177 mg (0,386 mMol) des Ketons von 1f) in 2,5 ml wasserfreiem THF tropfenweise der orangen Phosphinoxy-carbanionlösung zugesetzt. Das resultierende Gemisch wurde 1 Stunde bei -78°C gerührt. Es wurde dann mit 3 ml eines 1:1 (v:v) Gemisches von 2N Kaliumnatriumtartrat und 2N Kaliumcarbonat behandelt und, nach Aufwärmen auf Raumtemperatur, mit Wasser verdünnt und mit Aethylacetat extrahiert. Die vereinigten organischen Schichten wurden mit Salzwasser gewaschen, getrocknet und zur Trockene eingedampft. Der Rückstand wurde durch Filtrieren durch Silicagel und Elution mit Hexan-Aethylacetat gereinigt, dann in 0,8 ml Methylenchlorid und 9 ml Methanol gelöst und bei Raumtemperatur über Nacht mit 3,5 g eines Kationenaustauscherharzes (AG 50W-X4) gerührt. Nach Filtrieren und Eindampfen der Lösungsmittel wurde der Rückstand in 5 ml THF gelöst und mit 0,650 ml einer 1 molaren Lösung von Tetrabutylammoniumfluorid in THF behandelt und während 1 Stunde gerührt. Es wurde dann mit 0,5 ml Wasser behandelt und mit Aethylacetat extrahiert. Die vereinigten organischen Phasen wurden mit Wasser gewaschen, getrocknet und zur Trockene eingedampft. Das rohe Produkt wurde durch Chromatographie auf Silicagel mit Hexan-Aethylacetat gereinigt. Man erhielt 181 mg (90% Ausbeute) reines 26,26,26,27,27,27-Hexafluor-1α,25-dihydroxy -$\Delta^{22}$-cholecalciferol als weisses, amorphes Pulfer, $[\alpha]^{25}_D$ = +13.9° (c 0,2 in Aethanol).

Beispiel 2

a) Nach der Methode des Beispiels le) wurden 45,0 mg [1R-[1ß(R*),3aα,4ß,7aß]]-Octahydro-1-[6,6,6-trifluor -5-hydroxy-5-trifluormethyl-1-methyl-3-hexenyl]-7a-methyl -1H-inden -4-ol in 42,0 mg [1R-[1ß-[1ß(R*),3aα,4ß,7aß]]-Octahydro-1-[6,6,6-trifluor-5-hydroxy-5-trifluormethyl-1-methyl-3-hexenyl]-7a-methyl-1H-inden-4-on übergeführt.

b) Nach der Methode des Beispiels 1f) wurden 42,0 mg des Ketons von 2a) in 35,5 mg [1R-[1ß(R*),3aα,4ß,7aß]]-Octahydro-1-[6,6,6-trifluor-5-trimethylsilyloxy-5-trifluormethyl-1-methyl-3-hexenyl]-7a-methyl-1H-inden-4-on übergeführt.

c) Nach der Methode des Beispiels 1g) wurden 35,5 mg des Ketons von 2b) in 18,8 mg 26,26,26,27,27,27-Hexafluor-1α,25-dihydroxy -$\Delta^{23}$-cholecalciferol, $[\alpha]^{25}_D$ = +14,2° (c 0,1 in Aethanol) übergeführt.

Beispiel A

| Komponente | | mg/Kapsel | | |
|---|---|---|---|---|
| 1. | $\Delta^{22}$- oder $\Delta^{23}$-Verbindung | 0,00010 | 0,00025 | 0,00050 |
| 2. | Polyäthylenglykol-400 | 200,00 | 200,00 | 200,00 |
| 3. | butyliertes Hydroxyanisol | 0,100 | 0,100 | 0,100 |
| 4. | Ascorbylpalmitat | 1,00 | 1,00 | 1,00 |

Die Komponenten 1, 3 und 4 werden unter Stickstoff in der Komponente 2 gelöst und in eine Kapsel abgefüllt.

Beispiel B

| Komponente | | | |
|---|---|---|---|
| 1. | $\Delta^{22}$- oder $\Delta^{23}$-Verbindung | 0,10 mg | 0,50 mg |
| 2. | 95% Aethanol-5% Wasser | 2,00 ml | 3,00 ml |

Die Komponente 1 wird unter Stickstoff in die Komponente 2 gelöst und intramuskular injiziert.

**Patentansprüche für die Vertragsstaaten: BE, CH, DE, FR, GB, IT, LI, LU, NL, SE**

1. Das $\Delta^{22}$- oder $\Delta^{23}$-26,26,26,27,27,27-Hexafluor-1α,25-dihydcroxycholecalciferol.

2. [1R-[1β(R*),3aα,4β,7aβ]]-Octahydro-1-[6,6,6-trifluor-5-trimethylsilyloxy-5-trifluormethyl-1-methyl-2-hexenyl]-7a-methyl-1H-inden-4-on oder [1R-[1β(R*),3aα,4β,7aβ]]-Octahydro-1-[6,6,6-trifluor-5-trimethylsilyloxy-5-trifluormethyl-1-methyl-3-hexenyl]-7a-methyl-1H-inden-4-on.

3. $\Delta^{22}$- oder $\Delta^{23}$-26,26,26,27,27,27-Hexafluor-1α,25-dihydroxycholecalciferol als therapeutisch aktive Substanz, insbesondere mit Vitamin D-Aktivität.

4. Pharmazeutisches Präparat enthaltend $\Delta^{22}$- oder $\Delta^{23}$-26,26,26,27,27,27-Hexafluor-1α,25-dihydroxycholecalciferol als Wirksubstanz.

5. Verfahren zur Herstellung des $\Delta^{22}$- oder $\Delta^{23}$-26,26,26,27,27,27-Hexafluor-1α,25-dihydroxycholecalciferols, dadurch gekennzeichnet, daß man [1R[1β(R*),-3aα,4β,7aβ]]-Octahydro-1-[6,6,6-trifluor-5-trimethylsilyloxy-5-trifluormethyl-1-methyl-2-hexenyl]-7a-methyl-1H-inden-4-on bzw. [1R-[1β(R*),-3aα,4β,7aβ]]-Octahydro-1-[6,6,6-trifluor-5-trimethylsilyloxy-5-trifluormethyl-1-methyl-3-hexenyl]-7a-methyl-1H-inden-4-on mit [3S-(3α,5β,Z)]-2[2-Methylen-3,5-bis[(1,1-dimethyläthyl)-dimethylsilyloxy]cyclohexyliden]äthyldiphenylphosphinoxid behandelt und die Silylschutzgruppen abspaltet.

**Patentansprüche für den Vertragsstaat: AT**

1. Verfahren zur Herstellung des $\Delta^{22}$- oder $\Delta^{23}$-26,26,26,27,27,27-Hexafluor-1α,25-dihydroxycholecalciferols, dadurch gekennzeichnet, daß man [1R-[1β(R*),-3aα,4β,7aβ]]-Octahydro-1-[6,6,6-trifluor-5-trimethylsilyloxy-5-trifluormethyl-1-methyl-2-hexenyl]-7a-methyl-1-H-inden-4-on bzw. [1R-[1β(R*),3aα,-4β,7aβ]]-Octahydro-1-[6,6,6-trifluor-5-trimethylsilyloxy-5-trifluormethyl-1-methyl-3-hexenyl]-7a-methyl-1H-inden-4-on mit [3S-(3α,5β,Z)]-2-[2-Methylen-3,5-bis[(1,1-dimethyläthyl)-dimethylsilyloxy]-cyclohexyliden]äthyldiphenylphosphinoxid behandelt und die Silylschutzgruppen abspaltet.

2. Verwendung des $\Delta^{22}$- oder $\Delta^{23}$-26,26,26,27,27,27-Hexafluor-1α-25-dihydroxycholecalciferols zur Herstellung eines pharmateutischen Präparats mit Vitamin D-Aktivität.

**Claims for the Contracting States: BE, CH, DE, FR, GB, IT, LI, LU, NL, SE**

1. The $\Delta^{22}$- or $\Delta^{23}$-26,26,26,27,27,27-hexafluoro-1α,25-dihydroxycholecalciferol.

2. [1R-[1β(R*),3aα,4β,7aβ]]-Octahydro-1-[6,6,6-trifluoro-5-trimethylsilyloxy-5-trifluoromethyl-1-methyl-2-hexenyl]-7a-methyl-1H-inden-4-one or [1R-[1β(R*),3aα,4β,7aβ]]-octahydro-1-[6,6,6-trifluoro-5-trimethylsilyloxy-5-trifluoromethyl-1-methyl-3-hexenyal]-7a-methyl-1H-inden-4-one.

3. $\Delta^{22}$- or $\Delta^{23}$-26,26,26,27,27,27-Hexafluoro-1α,25-dihydroxycholecalciferol as a therapeutically active substance, especially with vitamin D activity.

4. A pharmaceutical preparation containing $\Delta^{22}$- or $\Delta^{23}$-26,26,26,27,27,27-hexafluoro-1α,25-dihydroxycholecalciferol as the active substance.

5. A process for the preparation of $\Delta^{22}$- or $\Delta^{23}$-26,26,26,27,27,27-hexafluoro-1α,25-dihydroxycholecalciferol, characterized by treating [1R-[1β(R*),3aα,4β,7aβ]]-octahydro-1-[6,6,6-trifluoro-5-trimethylsilyloxy-5-trifluoromethyl-1-methyl-2-hexenyl]-7a-methyl-1H-inden-4-one or [1R-[1β(R*),3aα,4β,-7aβ]]-octahydro-1-[6,6,6-trifluoro-5-trimethylsilyloxy-5-trifluoromethyl-1-methyl-3-hexenyl]-7a-methyl-1H-inden-4-one, respectively, with [3S-(3α,5β,Z)]-2-[2-methylene-3,5-bis[1,1-dimethylethyl)dimethylsilyloxy]cyclohexylidene]-ethyldiphenylphosphine oxide and cleaving off the silyl protecting groups.

**Claims for the Contracting State: AT**

1. A process for the preparation of $\Delta^{22}$- or $\Delta^{23}$-26,26,26,27,27,27-hexafluoro-1α,25-dihydroxycholecalciferol, characterized by treating [1R[1β(R*),3aα,4b,7aβ]]-octahydro-1-[6,6,6-trifluoro-5-trimethylsilyloxy-5-trifluoromethyl-1-methyl-2-hexenyl]-7a-methyl-1H-inden-4-one or [1R-[1β(R*),3aα,4β,7aβ]]-octahydro-1-[6,6,6-trifluoro-5-trimethylsilyloxy-5-trifluoromethyl-1-methyl-3-hexenyl]7a-methyl-1H-inden-4-one, respectively, with [3S-(3α,5β,Z)]-2-[2-methylene-3,5-bis[(1,1-dimethylethyl)dimethylsilyloxy]cyclohexylidene]-ethyldiphenylphosphine oxide and cleaving off the silyl protecting groups.

2. The use of $\Delta^{22}$- or $\Delta^{23}$-26,26,26,27,27,27-hexafluoro-1α,25-dihydroxycholecalciferol for the manufacture of a pharmaceutical preparation with vitamin D activity.

**Revendications pour les Etats contractants: BE, CH, DE, FR, GB, IT, LI, LU, NL, SE**

1. Le $\Delta^{22}$- ou $\Delta^{23}$-26,26,26,27,27,27-hexafluoro-1α,25-dihydroxycholécalciférol.

2. [1R-[1β(R*),3aα,4β,7aβ]]-octahydro-1-[6,6,6-trifluoro-5-triméthylsilyloxy-5-trifluorométhyl-1-méthyl-2-hexényl]-7a-méthyl-1H-indén-4-one ou [1R-[1β(R*),3aα,4β,7aβ]]-octahydro-1-[6,6,6-trifluoro-5-triméthylsilyloxy-5-trifluorométhyl-1-méthyl-3-hexényl]-7a-méthyl-1H-indén-4-one.

3. $\Delta^{22}$- ou $\Delta^{23}$-26,26,26,27,27,27-hexafluoro-1α,25-dihydroxycholécalciférol comme substance thérapeutiquement active, en particulier à activité de vitamine D.

4. Préparation pharmaceutique contenant le $\Delta^{22}$- ou $\Delta^{23}$-26,26,26,27,27,27-hexafluoro-1α,25-dihydroxycholécalciférol comme substance active.

5. Procédé de préparation du $\Delta^{22}$- ou $\Delta^{23}$-26,26,26,27,27,27-hexafluoro-1α,25-dihydrocycholécalciférol, caractérisé en ce qu'on traite la [1R-[1β(R*)-3aα,4β,7aβ]]-octahydro-1-[6,6,6-trifluoro-5-triméthylsilyloxy-5-trifluorométhyl-1-méthyl-2-hexényl]-7a-méthyl-1H-indén-4-one ou la [1R-[1β(R*),3aα,4β-7aβ]]-octahydro-1-[6,6,6-trifluoro-5-triméthylsilyloxy-5-trifluorométhyl-1-méthyl-3-hexényl]-7a-méthyl-1H-indén-4-one avec le [3S-(3α,5β,Z)]-2-[2-méthylène-3,5-bis-[(1,1-diméthyléthyl)-diméthylsilyloxy]-cyclohexylidène]-éthyldiphénylphosphinoxyde et en ce que les groupes protecteurs silyle sont détachés.

**Revendications pour l'Etat contractant: AT**

1. Procédé de préparation de $\Delta^{22}$- ou $\Delta^{23}$-26,26,26,27,27,27-hexafluoro-1α,25-dihydroxycholécalciférol, caractérisé en ce qu'on traite la [1R-[1β(R*),3aα,4β,7aβ]]-octahydro-1-[6,6,6-trifluoro-5-triméthylsilyloxy-5-trifluorométhyl-1-méthyl-2-hexényl]-7a-méthyl-1H-indén-4-one ou la [1R-[1β(R*),3aα,4β,-7aβ]]-octahydro-1-[6,6,6-trifluoro-5-triméthylsilyloxy-5-trifluorométhyl-1-méthyl-3-hexényl]-7a-méthyl-1H-indén-4-one avec le [3S-(3α,5β,Z)]-2-]-2-méthylène-3,5-bis-[1,1-diméthyléthyl)-diméthylsilyloxy]-cyclohexylidène]-éthyldiphénylphosphinoxyde et en ce qu'on sépare les groupes protecteurs de silyle.

2. Application du $\Delta^{22}$- ou $\Delta^{23}$-26,26,26,27,27,27-hexafluoro-1α,25-dihydroxycholécalciférol à la préparation d'une préparation pharmaceutique à activité de vitamine D.